# EUROPEAN PATENT APPLICATION

(11) **EP 2 638 855 A1**
(43) Date of publication of application: **18.09.2013**
(21) Application number: 10859585.1
(22) Date of filing: 08.11.2010
(51) Int. Cl.: A61B 5/18, A61B 5/11

(54) **SLEEP STATE ESTIMATION DEVICE**

(71) Applicant: TOYOTA JIDOSHA KABUSHIKI KAISHA, Toyota-shi, Aichi-ken, 471-8571 (JP)
(72) Inventor: SHIGETO, Kazuhide, Toyota-shi Aichi 471-8571 (JP)
(74) Representative: Winter, Brandl, Fürniss, Hübner, Röss, Kaiser, Polte - Partnerschaft
(86) International application number: PCT/JP2010/069847
(87) International publication number: WO 2012/063308

(57) **Abstract**

Since a change in a heart beat is dominated by the autonomic nerve which originates in the brain stem, it is difficult for an object person to control the heart beat at his or her own will. Therefore, frequency analysis, such as fine fluctuation analysis, is needed in order to estimate the body motion of the object person and is not suitable for instant processing. In contrast, according to this embodiment, an arithmetic unit 30 of a sleep state estimation device 1 estimates the state of the object person on the basis of the identity of each cycle of a breathing waveform of an object person M as the feature amount of the breathing waveform. The object person M can control a change in breathing at his or her own will. Therefore, when the state of the object person M is estimated on the basis of the identity of each cycle of the breathing waveform of the object person M, it is possible to easily classify the body motions of the object person M in detail and easily improve the accuracy of estimating the state of the object person M, such as the depth of sleep or the body motion.

## Description

### SLEEP STATE ESTIMATION DEVICE

The present invention relates to a sleep state estimation device, and more particularly, to a sleep state estimation device that estimates the state of an object person.

### Background Art

A device has been proposed which estimates the state of an object person during sleep to estimate the quality of sleep of the object person, such as a person with sleep apnea syndrome. According to the device, it is possible to perform various kinds of processes for the object person according to the quality of sleep of the object person. For example, Patent Literature 1 discloses a device including a staying-in-bed determination unit which determines staying in bed when the number of heart beats of the sleeping person is greater than a threshold value for determining whether a person stays in bed and determines leaving a bed when the number of heart beats is less than the threshold value for determining whether a person stays in bed. The determination result of whether the person stays in bed or leaves a bed is stored in a memory. A determination error detection unit determines that there is an error in the determination by the staying-in-bed determination unit when the kurtosis Kw of the frequency distribution of the number of heart beats is greater than a predetermined value in a section in which the staying-in-bed determination unit determines staying in bed, or when the kurtosis Kw of the frequency distribution of the number of heart beats is equal to or less than the predetermined value in a section in which the staying-in-bed determination section determines leaving a bed.

### Citation List

### Patent Literature

[Patent Literature 1] Japanese Unexamined Patent Application Publication No. 2010-88725

### Summary of Invention

### Technical Problem

However, the above-mentioned technique cannot accurately estimate the staying or leaving of a person in or from the bed. In the above-mentioned technique, it is determined whether a person stays in or leaves the bed with reference to a database using the kurtosis of the frequency distribution of the number of heart beats, which is a statistical value, in a given section. However, the above-mentioned technique cannot determine a body motion in a short time from the statistical value. For example, the above-mentioned technique does not distinguish an involuntary body motion from a voluntary body motion. In addition, the above-mentioned technique cannot specify the kind of body motion. For example, the above-mentioned technique does not distinguish intentional breathing such as breathing during a conversation or a deep breath. Furthermore, in the above-mentioned technique, a body motion being continuously made, for example, the unconscious shaking motion of the object person, is not distinguished from a normal body motion.

The invention has been made in view of the above-mentioned problems and an object of the invention is to provide a sleep state estimation device which can easily classify the body motions of an object person in detail and easily improve the accuracy of estimating the state of the object person, such as the depth of sleep or a body motion.

### Solution to Problem

According to an aspect of the invention, a sleep state estimation device includes a state estimation unit that estimates a state of an object person on the basis of identity of each cycle of a breathing waveform of the object person as a feature amount of the breathing waveform.

Since a change in a heart beat is dominated by the autonomic nerve which originates in the brain stem, it is difficult for the object person to control the heart beat at his or her own will. Therefore, frequency analysis, such as fine fluctuation analysis, is needed in order to estimate the body motion of the object person and the technique is not suitable for instant processing. In contrast, according to this structure, the state estimation unit estimates the state of the object person on the basis of the identity of each cycle of the breathing waveform of the object person as the feature amount of the breathing waveform. The object person can control a change in breathing at his or her own will. Therefore, when the state of the object person is estimated on the basis of the identity of each cycle of the breathing waveform of the object person, it is possible to easily classify the body motions of the object person in detail and easily improve the accuracy of estimating the state of the object person, such as the depth of sleep or a body motion.

In this case, the state estimation unit may estimate the state of the object person on the basis of at least one of reproducibility, which is a fluctuation in a minimum value in each cycle of the breathing waveform, and autocorrelation, which is identity between a waveform which is shifted from the breathing waveform by an arbitrary period of time and the original breathing waveform as the identity of each cycle of the breathing waveform of the object person.

According to this structure, the state estimation unit estimates the state of the object person on the basis of at least one of the reproducibility, which is a fluctuation in the minimum value in each cycle of the breathing waveform, and the autocorrelation, which is the identity between the waveform which is shifted from the breathing waveform by an arbitrary period of time and the original breathing waveform as the identity of each cycle of the breathing waveform of the object person. Therefore, it is possible to improve the accuracy of estimating the state of the object person, such as the depth of sleep or a body motion, with a simple process.

In this case, the state estimation unit may estimate the state of the object person on the basis of a cycle, an amplitude, the autocorrelation, and the reproducibility of the breathing waveform as the feature amount of the breathing waveform of the object person.

According to this structure, the state estimation unit estimates the state of the object person on the basis of the cycle, the amplitude, the autocorrelation, and the reproducibility of the breathing waveform as the feature amount of the breathing waveform of the object person. Therefore, since four indexes, such as the cycle, amplitude, autocorrelation, and reproducibility of the breathing waveform, are combined with each other to estimate the state of the object person, it is possible to further improve the accuracy of estimating the state of the object person, such as the depth of sleep or a body motion.

In this case, the state estimation unit may presume that the object person is reseated when it is detected that the amplitude, the autocorrelation, and the reproducibility of the breathing waveform are changed as compared to those in a normal state of the object person.

According to this structure, when it is detected that the amplitude, autocorrelation, and reproducibility of the breathing waveform are changed as compared to those in the normal state of the object person, the state estimation unit presumes that the object person is reseated. The inventors found that, when the object person was reseated, the amplitude, autocorrelation, and reproducibility of the breathing waveform of the object person were changed. Therefore, when it is detected that the amplitude, autocorrelation, and reproducibility of the breathing waveform are changed as compared to those in the normal state of the object person, it is presumed that the object person is reseated. As a result, it is possible to accurately presume that the object person is reseated.

In addition, the state estimation unit may presume that the object person stretches hands upward when it is detected that the amplitude and the reproducibility of the breathing waveform are changed as compared to those in the normal state of the object person.

According to this structure, the state estimation unit presumes that the object person stretches hands upward when it is detected that the amplitude and reproducibility of the breathing waveform are changed as compared to those in the normal state of the object person. The inventors found that, when the object person stretched the hands upward, the amplitude and reproducibility of the breathing waveform of the object person were changed. Therefore, when it is detected that the amplitude and reproducibility of the breathing waveform are changed as compared to those in the normal state of the object person, it is presumed that the object person stretches the hands upward. As a result, it is possible to accurately presume that the object person stretches the hands upward.

The state estimation unit may presume that the object person has a conversation when it is detected that the autocorrelation and the reproducibility of the breathing waveform are changed as compared to those in the normal state of the object person.

When it is detected that the autocorrelation and reproducibility of the breathing waveform are changed as compared to those in the normal state of the object person, the state estimation unit presumes that the object person has a conversation. The inventors found that, when the object person had a conversation, the autocorrelation and reproducibility of the breathing waveform of the object person were changed. Therefore, when it is detected that the autocorrelation and reproducibility of the breathing waveform are changed as compared to those in the normal state of the object person, it is presumed that the object person has a conversation. As a result, it is possible to accurately presume that the object person has a conversation.

The state estimation unit may presume that the object person takes a deep breath when it is detected that the cycle and the amplitude of the breathing waveform are changed as compared to those in the normal state of the object person.

According to this structure, when it is detected that the cycle and amplitude of the breathing waveform are changed as compared to those in the normal state of the object person, the state estimation unit presumes that the object person takes a deep breath. The inventors found that, when the object person took a deep breath, the cycle and amplitude of the breathing waveform of the object person were changed. Therefore, when it is detected that the cycle and amplitude of the breathing waveform are changed as compared to those in the normal state of the object person, it is presumed that the object person takes a deep breath. As a result, it is possible to accurately presume that the object person takes a deep breath.

The state estimation unit may compare the feature amount of the breathing waveform with a threshold value which is set to each feature amount to estimate the state of the object person.

According to this structure, the state estimation unit compares the feature amounts of the breathing waveform with the threshold value which is set to each feature amount to estimate the state of the object person. Therefore, it is possible to estimate the state of the object person, such as the depth of sleep or a body motion, with a simple process.

In this case, the state estimation unit may set the threshold value to each feature amount of each object person.

According to this structure, the state estimation unit sets the threshold value to each feature amount of each object person. Therefore, it is possible to estimate the state of the object person according to the physical constitution or taste of each object person.

The state estimation unit may estimate the state of the object person in a vehicle and estimate the state of the object person while discriminating between a behavior of the vehicle and a body motion of the object person on the basis of acceleration of the vehicle.

According to this structure, the state estimation unit estimates the state of the object person in the vehicle and estimates the state of the object person while discriminating between the behavior of the vehicle and the body motion of the object person on the basis of the acceleration of the vehicle. Therefore, it is possible to accurately estimate the state of the object person in the vehicle while discriminating between the behavior of the vehicle and the body motion of the object person.

The state estimation unit may determine whether the breathing type of the object person is abdominal breathing or chest breathing from the breathing waveform of the object person to estimate the depth of sleep of the object person.

According to this structure, the state estimation unit determines whether the breathing type of the object person is abdominal breathing or chest breathing from the breathing waveform of the object person to estimate the depth of sleep of the object person. Therefore, since whether the breathing type is abdominal breathing or chest breathing is closely related with the depth of sleep of the object person, it is possible to improve the accuracy of estimating the depth of sleep.

### Advantageous Effects of Invention

According to the sleep state estimation device of the invention, it is possible to easily classify the body motions of the object person in detail and easily improve the accuracy of estimating the state of the object person, such as the depth of sleep or a body motion.

### Brief Description of Drawings

Fig. 1 is a block diagram illustrating the structure of a sleep device according to an embodiment.
Fig. 2 is a diagram illustrating a breathing sensor that is provided in a seat of the sleep device shown in Fig. 1.
Fig. 3 is a diagram illustrating breathing bands that are provided in the seat of the sleep device shown in Fig. 1.
Fig. 4 is a flowchart illustrating the outline of the operation of the sleep device according to the embodiment.
Fig. 5 is a flowchart illustrating the details of the operation of the sleep device according to the embodiment.
Fig. 6 is a diagram illustrating a method of measuring the reproducibility of a breathing waveform.
Fig. 7 is a diagram illustrating a method of measuring the amplitude of the breathing waveform.
Fig. 8 is a diagram illustrating a method of measuring the autocorrelation of the breathing waveform.
Fig. 9 is a table illustrating the cycle, amplitude, and reproducibility of the breathing waveform for each type of body motion and vehicle behavior.
Fig. 10 is a table illustrating indexes indicating characteristics appearing in each type of body motion among the cycle, amplitude, autocorrelation, and reproducibility of the breathing waveform.
Fig. 11 is a diagram illustrating an example in which the deep breath of an object person is detected by the feature amount of the breathing waveform.
Fig. 12 is a diagram illustrating an example in which the reseating of the object person is detected by the feature amount of the breathing waveform.
Fig. 13 is a diagram illustrating an example in which the braking of the vehicle is detected by the feature amount of the breathing waveform.
Fig. 14 is a diagram illustrating an example in which a vehicle lane change is detected by the feature amount of the breathing waveform.
Fig. 15 is a diagram illustrating a noise reduction effect by filtering using the breathing waveform.
Fig. 16 is a table illustrating the relationship among the depth of sleep, the distribution of measurement positions, the dispersion of measurement positions, and a breathing type.
Fig. 17 is a diagram illustrating the relationship among the breathing waveform, the breathing type, and the position detected by a breathing sensor.
Fig. 18 is a diagram illustrating the relationship between a change in sleep stage and the breathing type.
Fig. 19 is a graph illustrating the relationship between the change in sleep stage and the breathing type.
Fig. 20 is a diagram illustrating the change in sleep stage and a section in which the feature amount of the breathing waveform is calculated.
Fig. 21 is a diagram illustrating the relationship between the change in sleep stage and the breathing type.
Fig. 22 is a table illustrating the change in sleep stage and a theoretical change in the breathing type.
Fig. 23 is a graph illustrating the rate of change of the sleep stage between the sections shown in Fig. 20 in which the feature amount is calculated.
Fig. 24 is a table illustrating the rate of change of the sleep stage between the sections shown in Fig. 20 in which the feature amount is calculated.
Fig. 25 is a graph illustrating the value of a section P1/a section P2 shown in Fig. 20 each time the sleep stage changes in abdominal breathing shown in Fig. 24.
Fig. 26 is a graph illustrating average values when the depth of sleep increases from sleep stage 2 to sleep stage 3 as shown in Fig. 25 and when the depth of sleep decreases from sleep stage 3 to sleep stage 2.
Fig. 27 is a table illustrating a significant difference test assuming that two samples shown in Fig. 26 have equal variance.

### Description of Embodiments

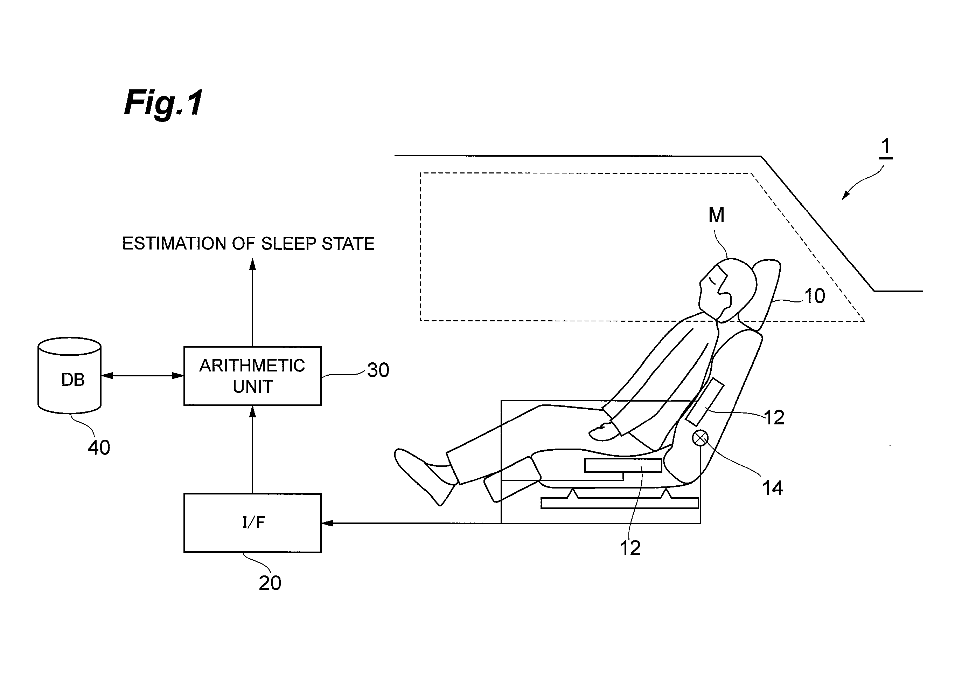

Hereinafter, a sleep state estimation device according to an embodiment of the invention will be described with reference to the accompanying drawings. The sleep state estimation device according to this embodiment is provided in, for example, a vehicle, estimates the sleep state of an object person, such as the depth of sleep or a body motion, and responds to situations using various application programs. As shown in Fig. 1, a sleep state estimation device 1 according to this embodiment includes a seat 10, a breathing sensor 12, an acceleration sensor 14, an 1/F 20, an arithmetic unit 30, and a DB 40.

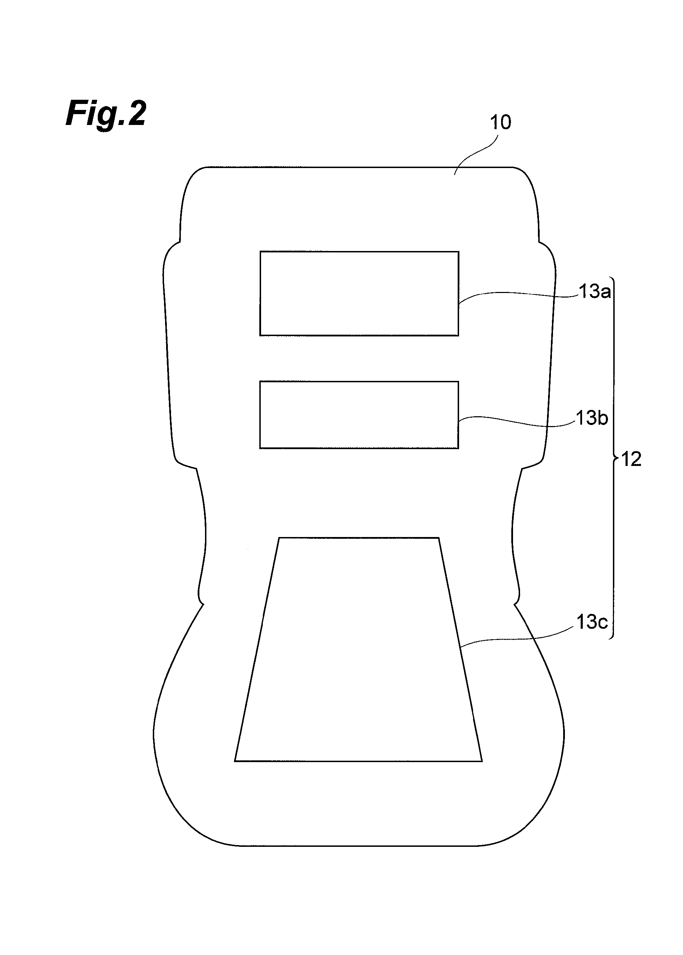

The breathing sensor 12 is a sensor which measures the breathing of an object person M in a non-invasive manner. Specifically, as shown in Figs. 1 and 2, the breathing sensor 12 includes a plurality of pressure sensors which are provided on the seating surface or the back of the seat 10. As shown in Fig. 2, the breathing sensor 12 includes a pressure sensor 13 a that measures the movement of the chest of the object person M, a pressure sensor 13b that measures the movement of the abdomen of the object person M, and a pressure sensor 13c that measures the pressure of the object person M against the seating surface, which are provided in the seat 10.

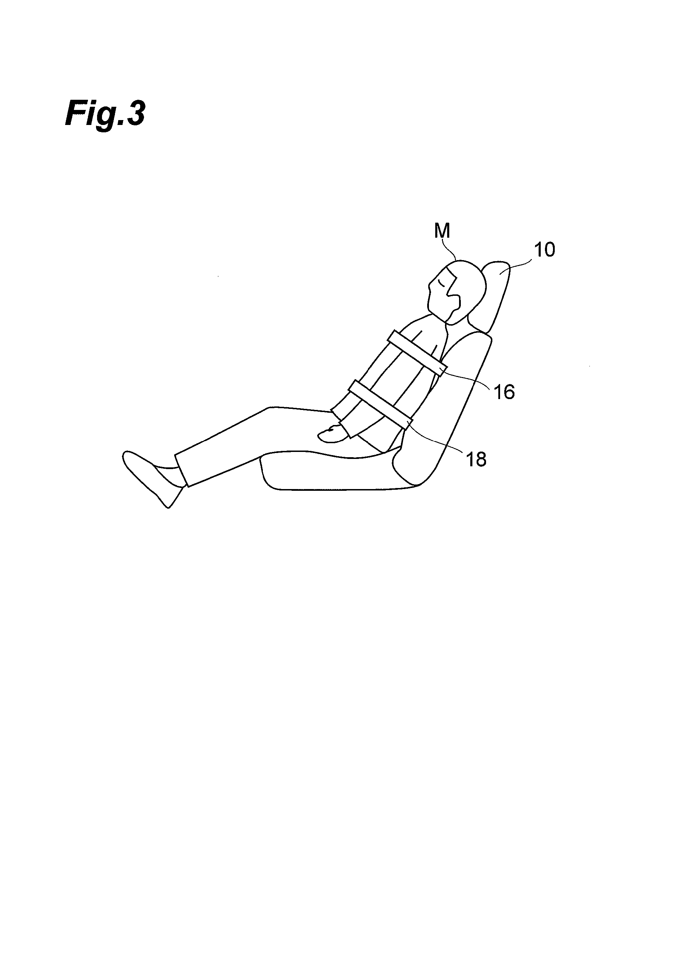

As shown in Fig. 3, the seat 10 may include breathing bands 16 and 18 that measure the breathing of the object person M. The breathing bands 16 and 18 are strain gauge bands capable of detecting the breathing of the object person M. The breathing band 16 detects the movement of the chest of the object person M. The breathing band 18 detects the movement of the abdomen of the object person M. In addition, the seat 10 may separately include a heart beat sensor or a brain wave sensor.

Returning to Fig. 1, the seat 10 includes an acceleration sensor 14 that detects the acceleration of the vehicle. The acceleration sensor 14 may be provided in the vehicle in advance. Alternatively, the acceleration sensor 14 may be provided in the sleep state estimation device 1.

The values detected by the breathing sensor 12 and the acceleration sensor 14 are processed by the arithmetic unit 30 through the 1/F 20. The arithmetic unit 30 estimates the sleep state of the object person M with reference to the DB 40 in which data for each object person M is recorded. The sleep state includes the depth of sleep of the object person M and information indicating whether body motions, such as the deep breathing of the object person M, conversation, reseating, the stretching of both hands in the upward direction, and the moving-up of one of the hips, are made.

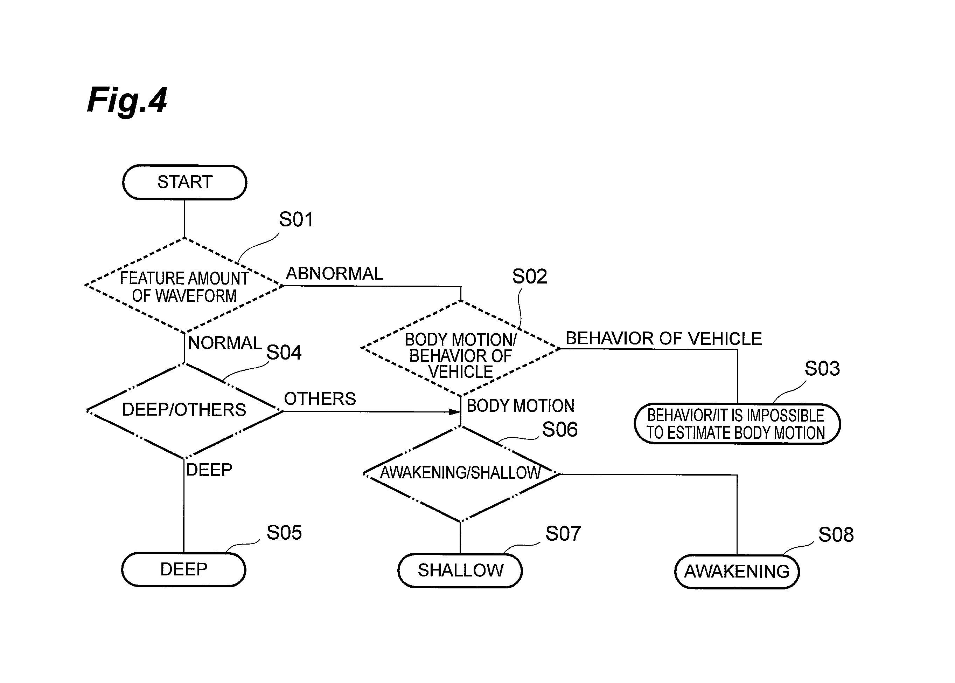

Next, the operation of the sleep state estimation device 1 according to this embodiment will be described. First, the outline of the operation will be described. As shown in Fig. 4, the arithmetic unit 30 of the sleep state estimation device 1 determines whether the feature amount (which will be described below) of a breathing waveform detected by, for example, the breathing sensor 12 is normal or abnormal (S01). When the feature amount of the breathing waveform is abnormal (S01), the arithmetic unit 30 determines whether the abnormal feature amount of the breathing waveform is caused by the body motion of the object person M, such as a deep breath, or the behavior of the vehicle, such as a braking operation of the vehicle, on the basis of the values detected by the breathing sensor 12 and the acceleration sensor 14 (S02). When the abnormal feature amount of the breathing waveform is caused by the behavior of the vehicle (S02), the arithmetic unit 30 determines that the abnormal feature amount of the breathing waveform is caused by the behavior of the vehicle and it is impossible to estimate the body motion of the object person M (S03). In Steps S01 to S03, a filtering process is performed using the output waveforms from the breathing sensor 12 and the acceleration sensor 14.

When the feature amount of the breathing waveform is normal (S01), the arithmetic unit 30 determines whether the object person M is in a deep sleep state or in states other than the deep sleep state (S04). When it is determined that the object person M is in the deep sleep state, the arithmetic unit 30 outputs information indicating that the object person M is in the deep sleep state (S04 and S05). When the feature amount of the breathing waveform is abnormal (S01) and the abnormal feature amount of the breathing waveform is caused by the body motion of the object person M (S02), or when the feature amount of the breathing waveform is normal (S01) and the object person M is in a state other than the deep sleep state (S04), the arithmetic unit 30 determines whether the object person M is in an awakening state or a shallow sleep state (S06). The arithmetic unit 30 outputs the determination result of whether the object person M is in the awakening state or in the shallow sleep state (S06 to S08). In Steps S04 to S08, the arithmetic unit 30 estimates the sleep state of the object person M with reference to learning data stored in the DB 40 or while storing new learning data in the DB 40.

In this embodiment, in Step S04, the arithmetic unit 30 may determine whether the object person M is awake or asleep. In this case, when it is determined that the object person M is asleep (S04), the arithmetic unit outputs the operation result indicating that the object person M is asleep in Step S05. When the feature amount of the breathing waveform is normal (S01) and the object person M is awake (S04), or when the feature amount of the breathing waveform is abnormal (S01) and the abnormal feature amount of the breathing waveform is caused by the body motion of the object person M (S02), the arithmetic unit 30 outputs the operation result indicating that the object person M is awake.

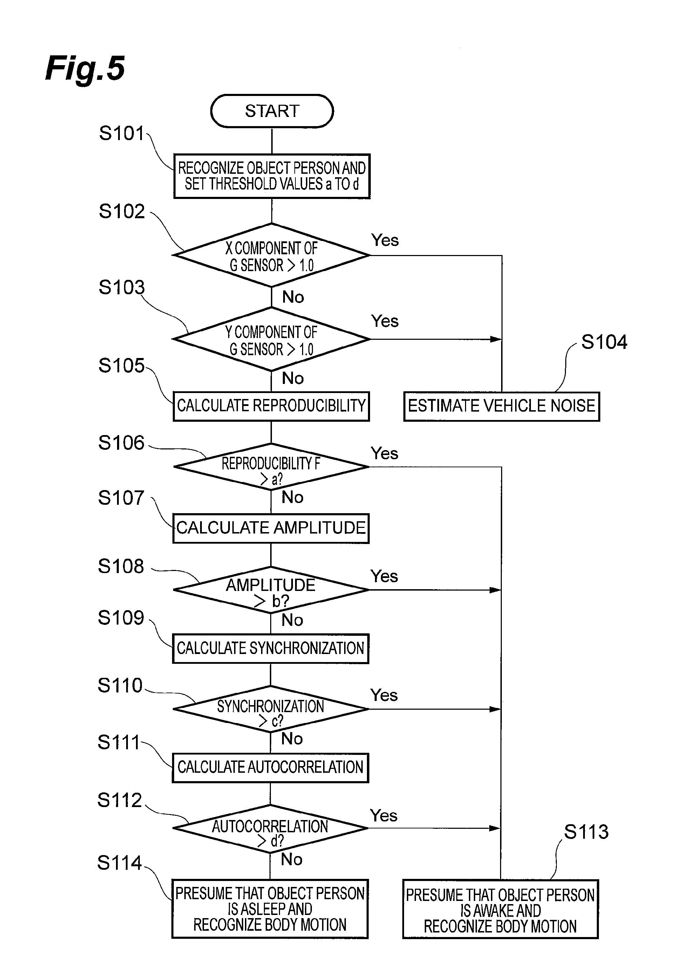

Next, the operation of the sleep state estimation device 1 according to this embodiment will be described in detail. As shown in Fig. 5, the arithmetic unit 30 recognizes the object person M and sets the threshold values a to d of reproducibility, an amplitude, a cycle, and autocorrelation which are the feature amounts of the breathing waveform, which will be described below (S101). The threshold values a to d are set to each object person M and are recorded in the DB 40.

The arithmetic unit 30 determines whether an X component (in the front-rear direction of the vehicle) of acceleration is greater than a threshold value of, for example, 1.0 m/s2 using the value detected by the acceleration sensor 14 (S102). The arithmetic unit 30 determines whether a Y component (the left-right direction of the vehicle) of the acceleration is greater than a threshold value of, for example, 1.0 m/s2 using the value detected by the acceleration sensor 14 (S103).

When either of the X component and the Y component of the acceleration is greater than a threshold value of, for example, 1.0 m/s2 (S102 and S103), the arithmetic unit 30 estimates the value detected by the breathing sensor 12 as vehicle noise (S 104). When neither of the X component nor the Y component of the acceleration is greater than a threshold value of, for example, 1.0 m/s2 (S102 and S103), the arithmetic unit 30 calculates reproducibility as the feature amount from the value detected by the breathing sensor 12 (S105).

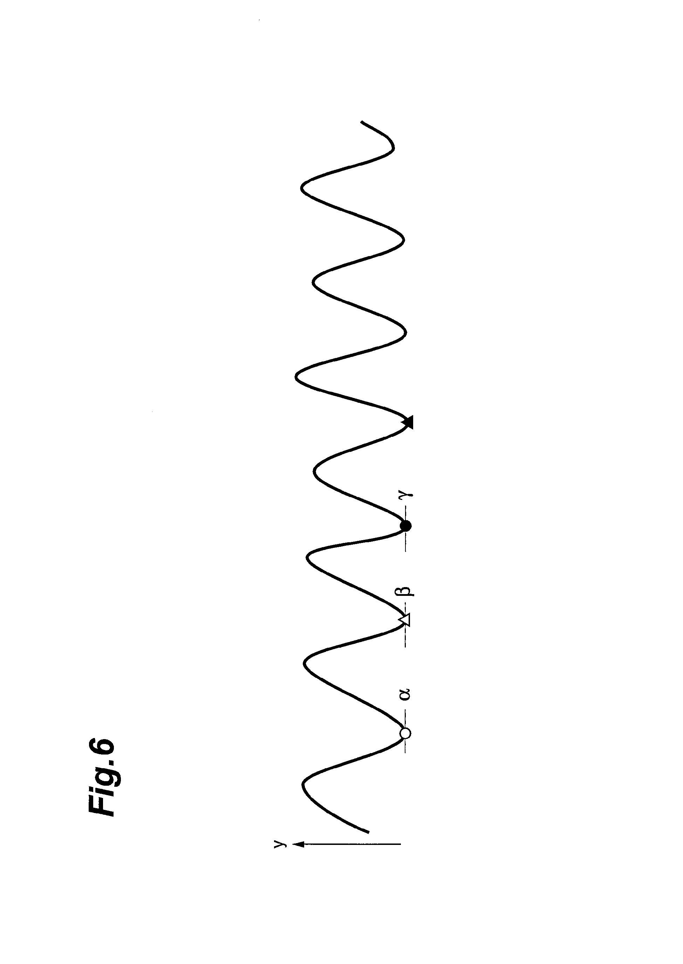

The reproducibility means a fluctuation in the minimum value in each cycle of the breathing waveform. Assuming that the breathing sensor 12 detects the breathing waveform shown in Fig. 6, the minimum value in each cycle of the breathing waveform is □, □, and □. An average value □ is calculated as follows: □ = {(□-□)+(□-□)}/2. Reproducibility F is calculated as follows: F = {(□-□-□)2+(□-□-□)2}1/2.

Returning to Fig. 5, the arithmetic unit 30 determines whether the reproducibility F is greater than the threshold value a (S106). When the reproducibility F is greater than the threshold value a (S106), the arithmetic unit 30 presumes that the object person M is awake and recognizes the body motion of the object person M as described below (S113).

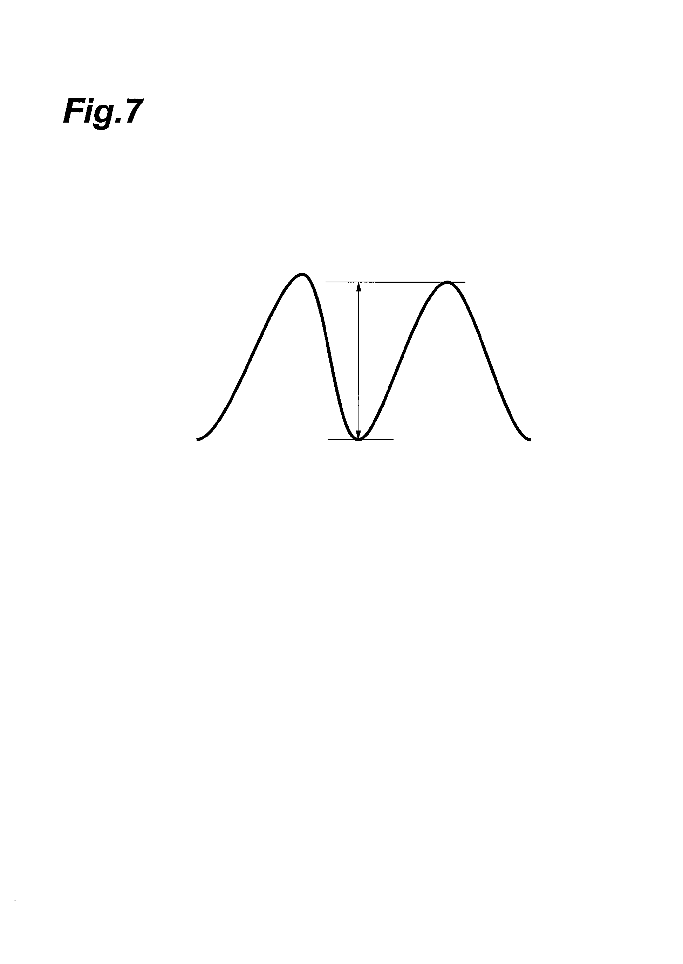

When the reproducibility F is equal to or less than the threshold value a (S 106), the arithmetic unit 30 calculates an amplitude as the feature amount from the value detected by the breathing sensor 12 (S107). As shown in Fig. 7, the amplitude of the breathing waveform is calculated from the difference between the minimum value and the maximum value of the breathing waveform in the previous cycle.

Returning to Fig. 5, the arithmetic unit 30 determines whether the amplitude is greater than the threshold value b (S108). When the amplitude is greater than the threshold value b (S 108), the arithmetic unit 30 presumes that the object person M is awake and recognizes the body motion of the object person M as described below (S 113).

When the reproducibility F is equal to or less than the threshold value a (S108), the arithmetic unit 30 calculates a cycle as the feature amount from the value detected by the breathing sensor 12 (S109). The arithmetic unit 30 determines whether the cycle is greater than the threshold value c (S110). When the cycle is greater than the threshold value c (S110), the arithmetic unit 30 presumes that the object person M is awake and recognizes the body motion of the object person M as described below (S 113).

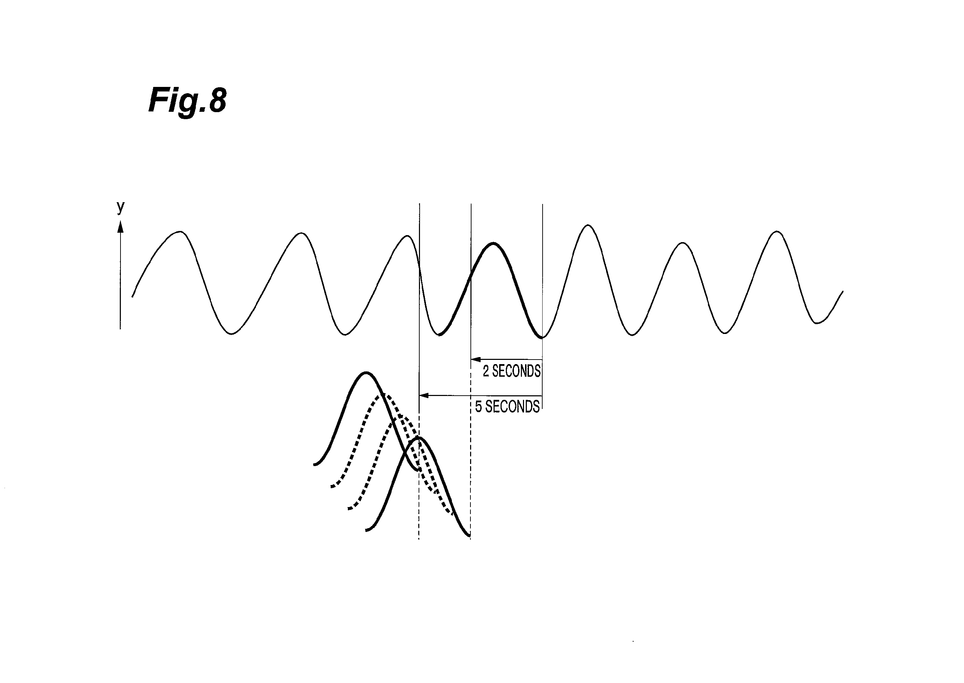

When the cycle is equal to or less than the threshold value c (S110), the arithmetic unit 30 calculates autocorrelation as the feature amount (S111). The term "autocorrelation" means the identity between a waveform which is shifted from the breathing waveform by an arbitrary period of time and the original breathing waveform. For example, as shown in Fig. 8, the breathing waveform detected by the breathing sensor 12 is shifted by 2 seconds, 5 seconds, or 1 to 3 cycles and the difference between the shifted waveform and the original breathing waveform is digitized to calculate the autocorrelation. For the autocorrelation, for example, the value of the difference between a waveform which is shifted from the breathing waveform by a given cycle and the original breathing waveform and the value of the difference between a waveform which is shifted from the breathing waveform by another cycle and the original breathing waveform are combined with each other and it is determined that the autocorrelation is changed when there is a difference between the values. In this way, it is possible to improve the accuracy of detection.

Returning to Fig. 5, the arithmetic unit 30 determines whether the autocorrelation is greater than the threshold value d (S112). When the autocorrelation is greater than the threshold value d (S 112), the arithmetic unit 30 presumes that the object person M is awake and recognizes the body motion of the object person M as described below (S 113). When the autocorrelation is equal to or less than the threshold value d (S112), the arithmetic unit 30 presumes that the object person M is asleep and recognizes the sleep stage and the body motion of the object person M as described below (S 114).

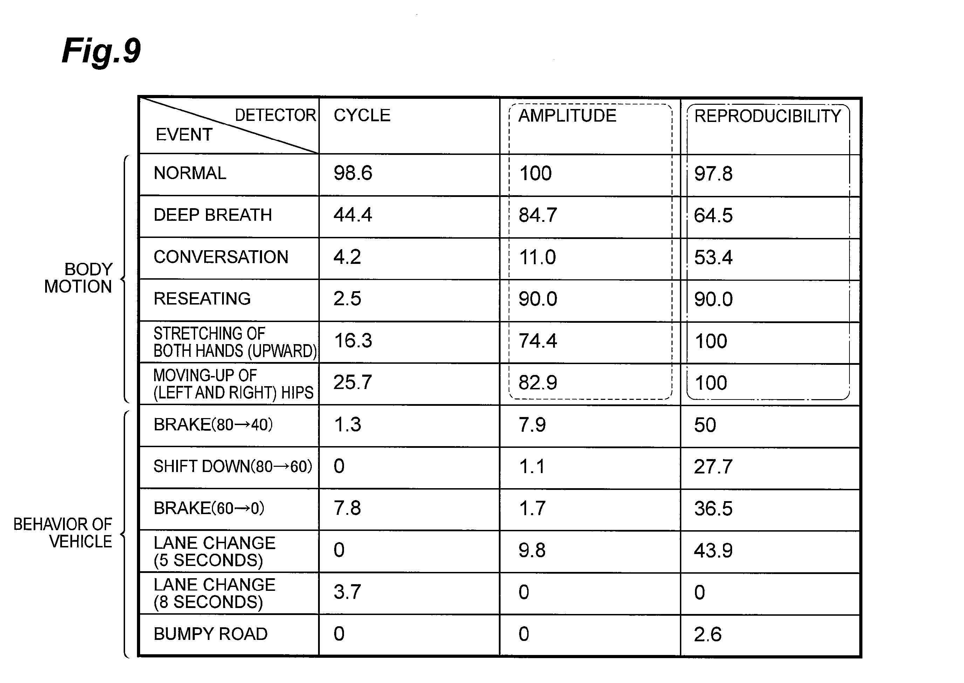

Next, a method of recognizing the body motion of the object person M will be described. The inventors found that the use of combinations of the amplitude, cycle, autocorrelation, and reproducibility of the breathing waveform of the object person M made it possible to estimate the body motion of the object person M with high probability. For example, as can be seen from Fig. 9, for example, the amplitude and reproducibility of the breathing waveform of the object person M greatly vary depending on the body motion of the object person M, such as a deep breath, or the behavior of the vehicle, such as braking.

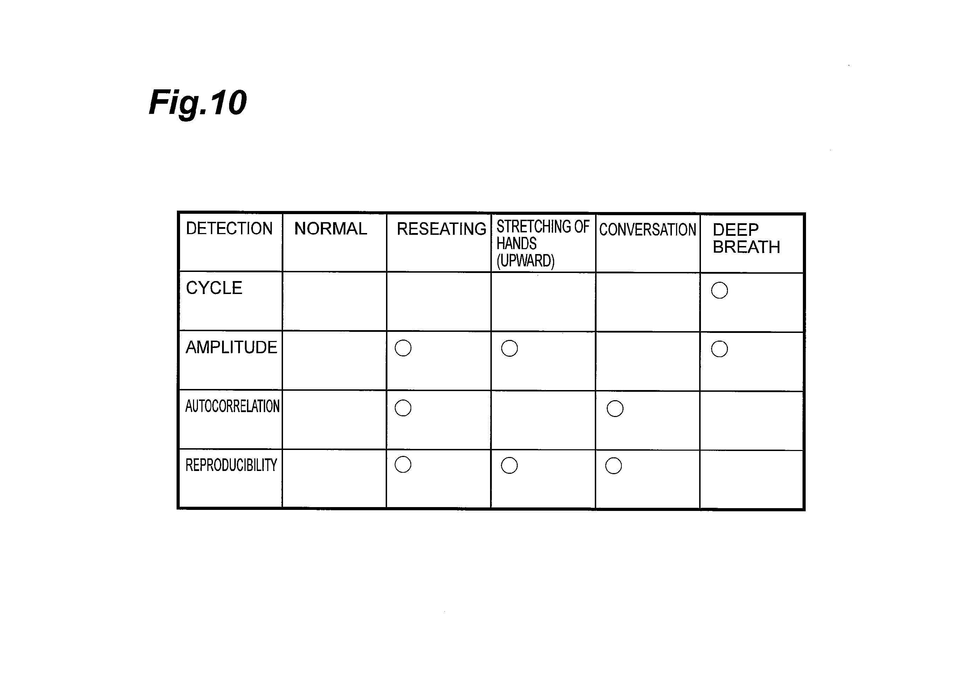

Therefore, in this embodiment, the body motion of the object person M is estimated by the combinations of the amplitude, cycle, autocorrelation, and reproducibility of the breathing waveform of the object person M. As shown in Fig. 10, when the object person M is reseated, the amplitude, autocorrelation, and reproducibility of the breathing waveform are changed. When the object person M stretches the hands upward, the amplitude and reproducibility of the breathing waveform are changed. When the object person M has a conversation, the autocorrelation and reproducibility of the breathing waveform are changed. When the object person M takes a deep breath, the cycle and amplitude of the breathing waveform are changed. In addition, the inventors found that, for the body motions of the object person M, such as the recrossing of the legs or yawning, the use of combinations of the feature amounts of the breathing waveform, such as the amplitude and the autocorrelation, made it possible to detect the body motions with high accuracy.

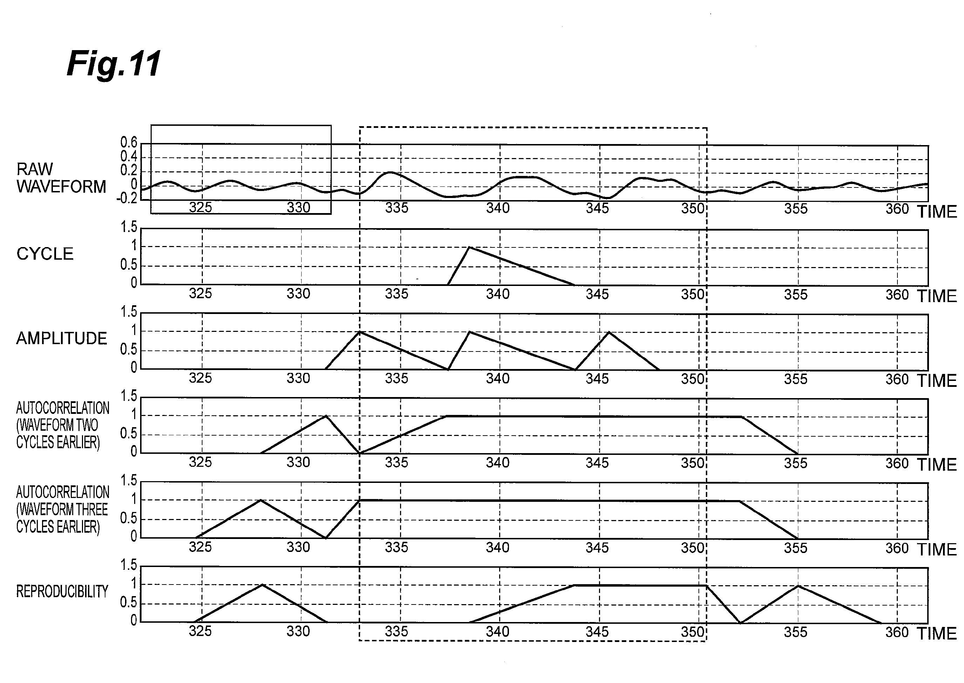

A change in the feature amounts of the breathing waveform when the object person M takes three deep breaths will be described with reference to Fig. 11. In Fig. 11, the raw waveform, cycle, and amplitude of the breathing waveform, the autocorrelation between the waveform two cycles earlier and the current breathing waveform, the autocorrelation between the waveform three cycles earlier and the current breathing waveform, and the reproducibility are sequentially shown from the upper side. For ease of comparison, the calculated values of the cycle, the amplitude, the autocorrelation, and the reproducibility are normalized, a value of 0 indicates that there is no change from a normal state, and a value greater than 0 indicates that there is a change from the normal state. When the normalized value is greater than 1, 1 is used as the upper limit. As can be seen from Fig. 11, during deep breathing which is represented by a dashed line, a change in the cycle and amplitude of the breathing waveform is more remarkable than that in the normal state which is represented by a solid line.

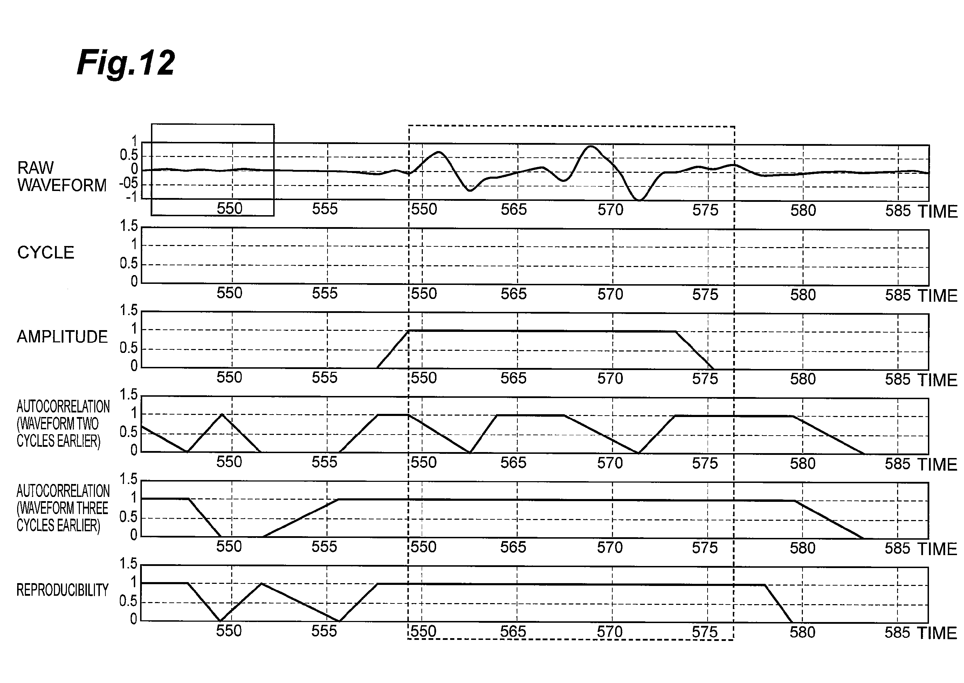

Similarly, a change in the feature amounts of the breathing waveform when the object person M is reseated two times will be described with reference to Fig. 12. As can be seen from Fig. 12, during reseating which is represented by a dashed line, a change in the amplitude, autocorrelation, and reproducibility of the breathing waveform is more remarkable than that in the normal state which is represented by a solid line. However, there is no remarkable change in the cycle of the breathing waveform.

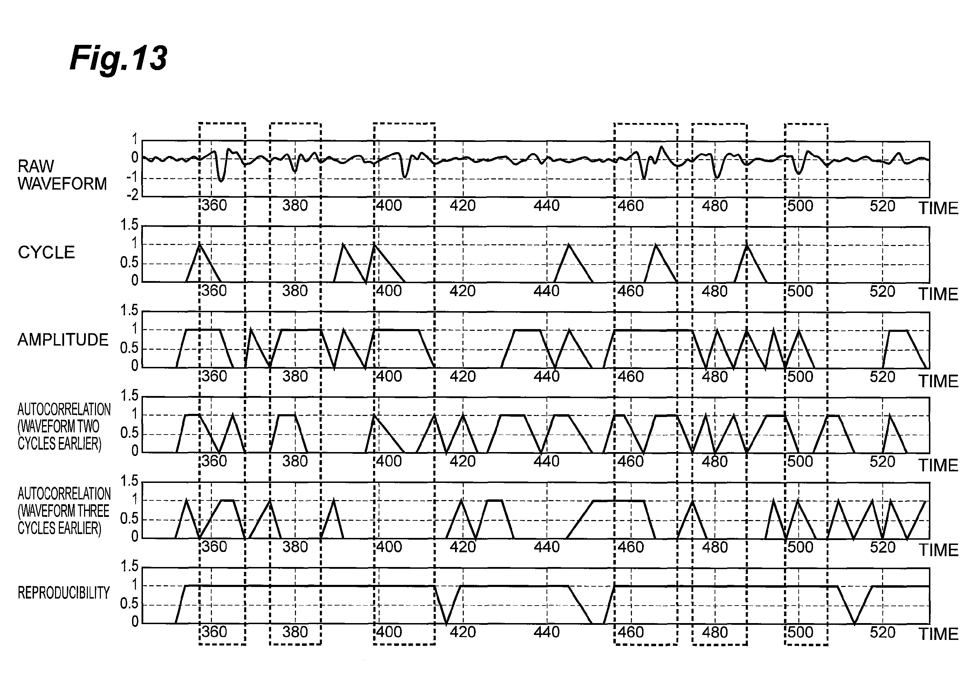

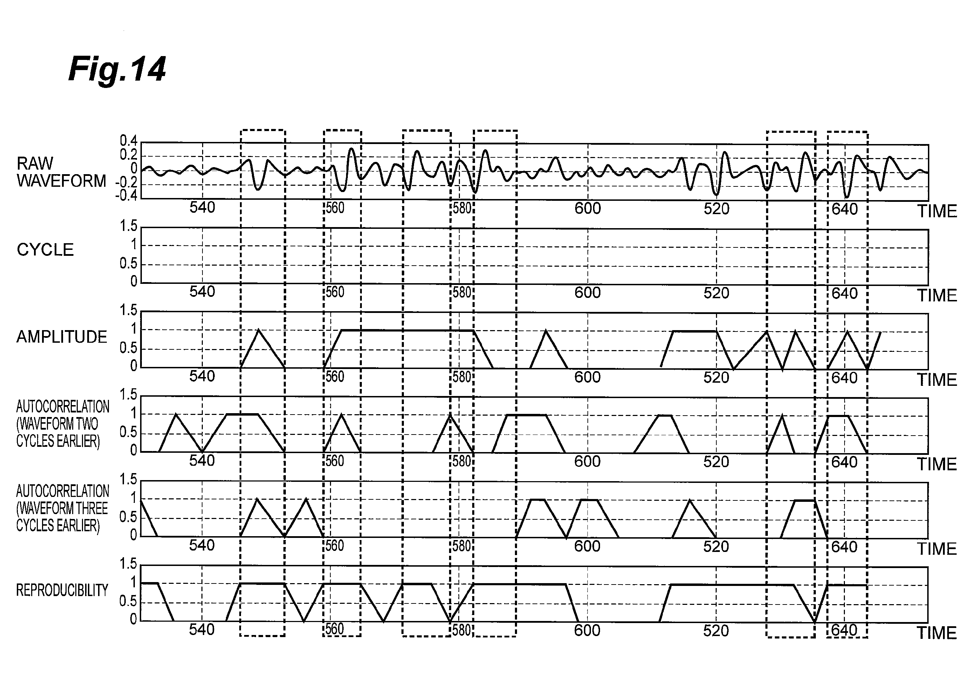

Figs. 13 and 14 show the breathing waveforms and the feature amounts thereof when an operation of braking the vehicle from a speed of 60 km/h to stop the vehicle is repeatedly performed six times and an operation of changing the lane at a speed of 60 km/h is repeatedly performed six times, respectively. As can be seen from Figs. 13 and 14, when the behavior of the vehicle changes, the breathing waveform of the object person M and the feature amounts thereof are changed. Therefore, when the sleep state estimation device 1 according to this embodiment is applied to the vehicle, it is possible to classify the body motions of the object person M, such as a deep breath, conversation, reseating, and the stretching (extension) of both hands, and classify the behaviors of the vehicle, such as the braking of the vehicle, a lane change, shifting down a gear, and traveling on a bumpy road.

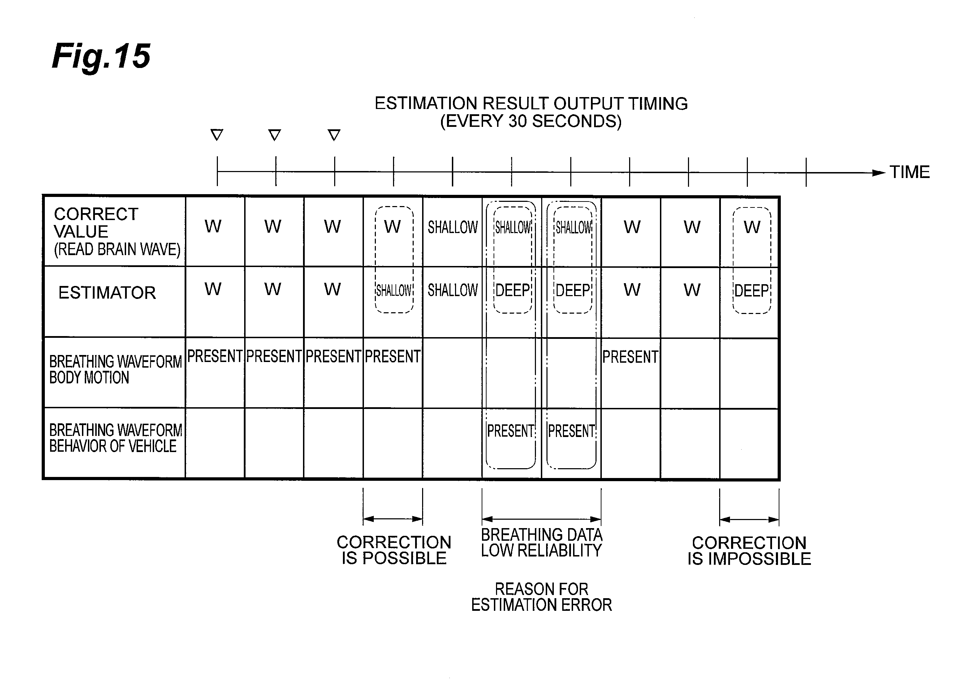

In this embodiment, in addition to the breathing waveform of the object person M and the feature amounts thereof, the value detected by the acceleration sensor 14 is used to remove noise. In this way, the accuracy of detecting the state of the object person M is improved. For example, as shown in Fig. 15, in some cases, even though the object person M is awake (W) in practice, the arithmetic unit 30 serving as an estimator outputs the wrong estimation result indicating that the depth of sleep of the object person M is small. However, in this case, when the body motion is estimated by the breathing waveform, it is possible to correct the estimation result since the possibility of the object person M being awake is high.

Alternatively, even when the arithmetic unit 30 serving as an estimator outputs the wrong estimation result indicating that the depth of sleep of the object person M is large even though the depth of sleep of the object person M is small in practice, the behavior of the vehicle is estimated by the breathing waveform. However, when the behavior of the vehicle is detected by the acceleration sensor 14, it is possible to determine that the reliability of the estimation result based on the breathing waveform which indicates that the depth of sleep is large is low, and the behavior of the vehicle detected by the acceleration sensor can be used to verify the reason for the wrong estimation result. When the body motion of the object person M or the behavior of the vehicle cannot be detected by the breathing waveform or the acceleration sensor 14, it may be difficult to correct the wrong estimation result. However, when the body motion of the object person M or the behavior of the vehicle is constantly detected, it is possible to correct the estimation result and improve the accuracy of estimation.

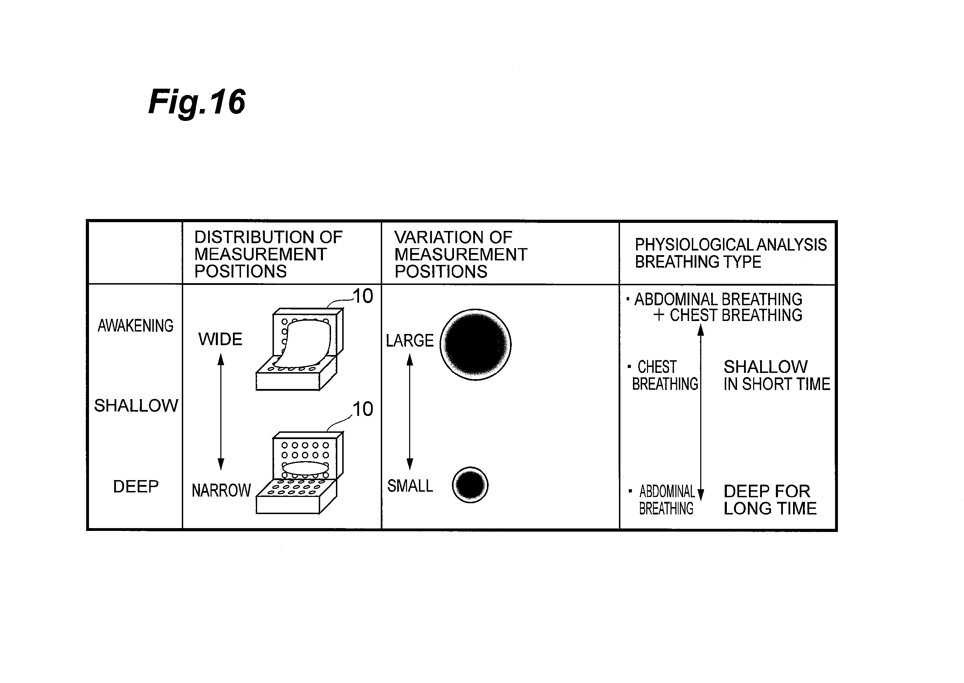

In this embodiment, a primary filter using the detection positions of a plurality of breathing sensors 12 provided in the seat 10 is provided to classify the sleep states mainly into a deep sleep state and a shallow sleep state. As shown in Fig. 16, as breathing characteristics during normal sleep, people generally breathe from the abdomen in the deep sleep state and generally breathe from the chest in the shallow sleep state. Therefore, in this embodiment, it is determined whether the breathing type of the object person M is abdominal breathing or chest breathing on the basis of the detection positions of the plurality of breathing sensors 12 provided in the seat 10 and the sleep state of the object person M is classified into the deep sleep state and the shallow sleep state with high accuracy. In addition, since the body motion of a person tends to be stabilized (remain calm) in a deep sleep stage, it is possible to disperse the measurement positions to estimate the depth of sleep.

The breathing types are mainly classified into chest breathing and abdominal breathing. In many cases, elements of the two breathing types are mixed with each other in an unconscious state. In general, the emotional state of the object person M is strongly related to breathing. When a person is strained physically and mentally, breathing is shallow and short. That is, when there is a sense of tension or a sense of unease, a person is likely to breathe from the chest in the unconscious state. On the contrary, during sleep, the tension of a person is reduced and the person is in a relaxed state. Therefore, in the deep sleep state, abdominal breathing is dominant. The amount of air inhaled and exhaled in an abdominal breath is several times more than that of air inhaled and exhaled in a chest breath. For example, while the amount of air in the chest breathing is 500 ml, the amount of air in the abdominal breathing is a maximum of 2000 ml. When the above is considered, it is proved that the frequency of breathing, which is one of the observable physiological indexes, in the deep sleep state is lower than that in an active state or in the shallow sleep state.

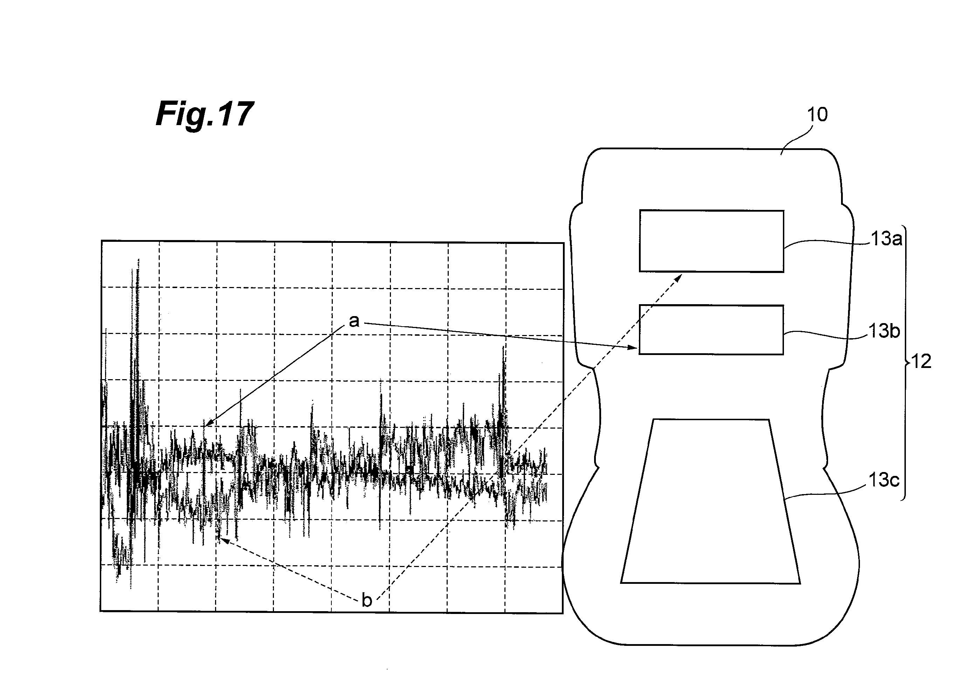

In this embodiment, as shown in Fig. 17, the pressure sensors 13a and 13b of the breathing sensor 12 are arranged at least at the upper and lower ends of the seat 10 to measure the movement of the chest and abdomen of the object person M and the sleep states are classified into the deep sleep state and the other states on the basis of a change in the breathing type of the object person M, that is, abdominal breathing a and chest breathing b. In this embodiment, the breathing bands 16 and 18 shown in Fig. 3 may be used. Specifically, the arithmetic unit 30 of the sleep state estimation device 1 calculates the difference between the amplitudes or phases of abdominal breathing and chest breathing for one breath or each breath, averages the difference for 30 seconds, calculates a coefficient of variation in a processing section of 30 seconds, compares the magnitudes between abdominal breathing and chest breathing, and performs differentiation and integration in the processing section to calculate vectors.

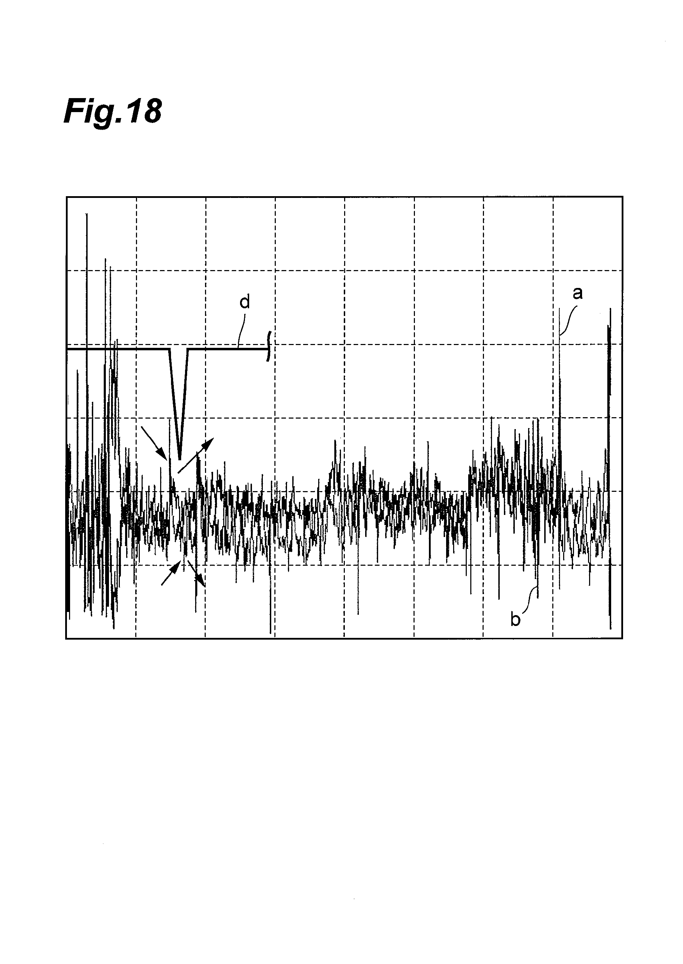

Next, the relationship between a change in the sleep stage and the breathing type will be described. Fig. 18 shows the abdominal breathing a, the chest breathing b, and the sleep stage d of the object person. As can be seen Fig. 18, whenever the sleep stage d changes, the abdominal breathing a counteracts the chest breathing b.

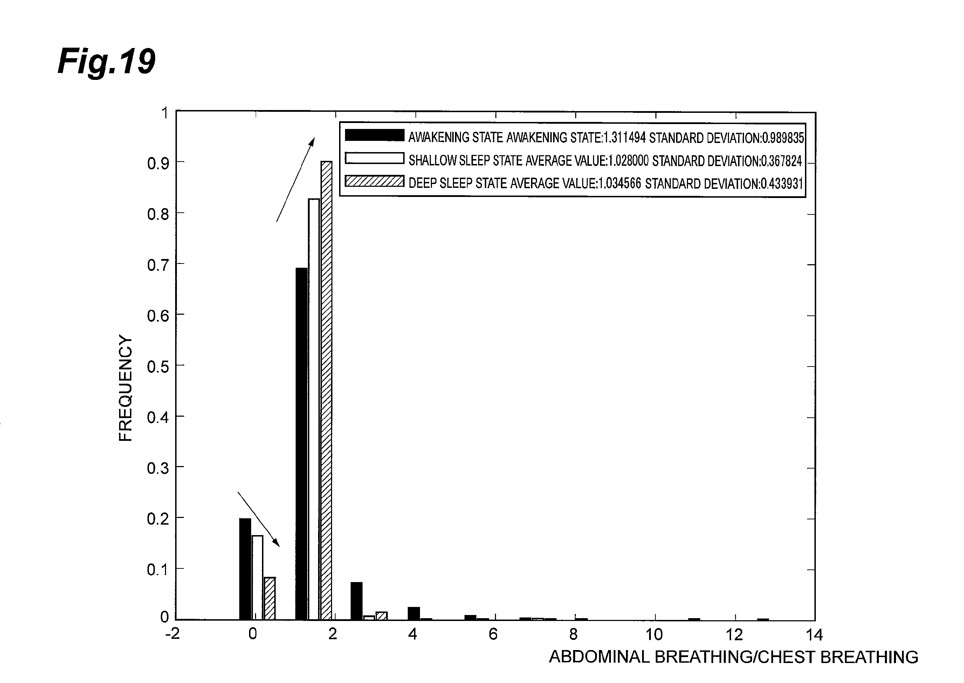

As such, it is ascertained that a change in the sleep stage is correlated with a change in the breathing type in the previous and next sections. However, when the difference in the value detected by the breathing sensor 12 is used to calculate the change, it is expected that the value will not be stable due to, for example, a change in the breathing sensor 12 or a change in output gain. Therefore, in this embodiment, the ratio is used to respond to the change. For example, as can be seen from Fig. 19 in which the horizontal axis indicates the ratio (abdomen/chest) of abdominal breathing to chest breathing and the vertical axis indicates the frequency of the ratio, the ratio of chest breathing is high in the awakening state and the ratio of abdominal breathing is high in the deep sleep state. In addition, as can be seen from Fig. 19, the standard deviation between abdominal breathing and chest breathing is large in the awakening state and the standard deviation between abdominal breathing and chest breathing is small in the sleep state.

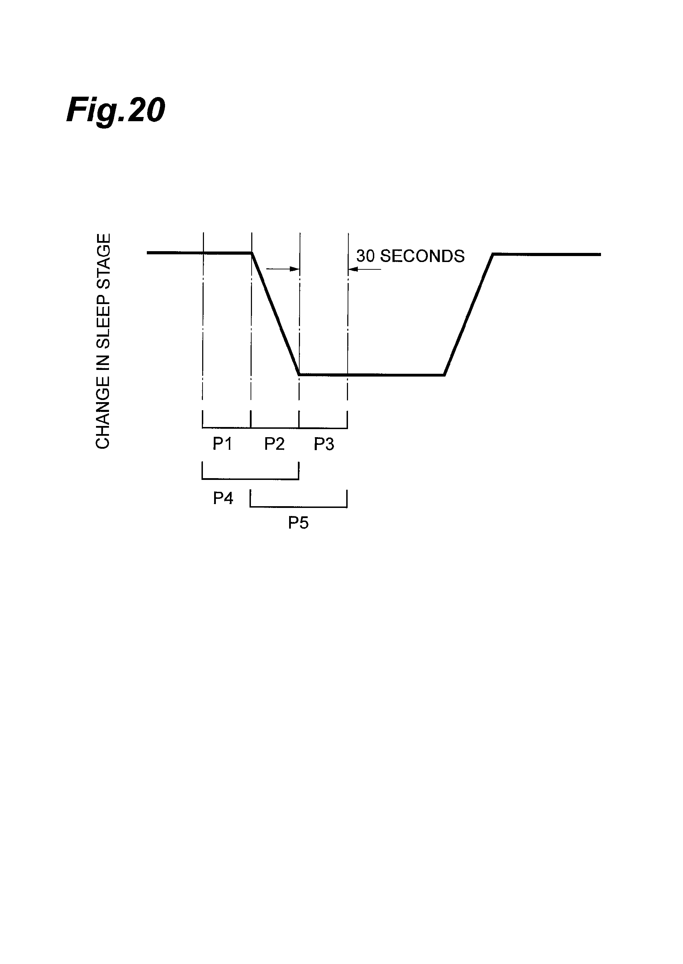

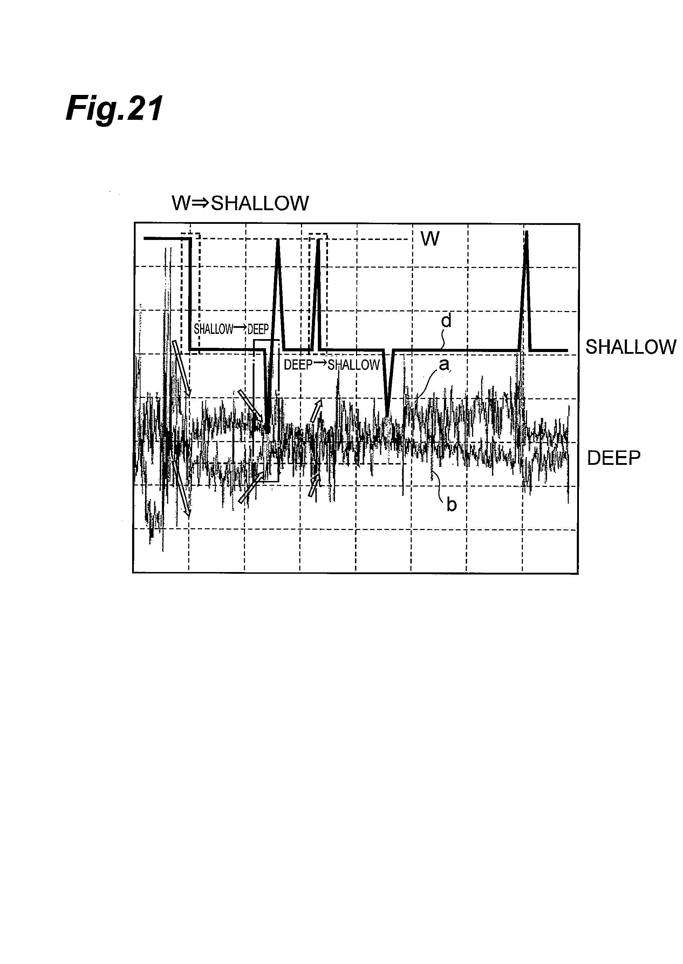

Here, the section shown in Fig. 20 is defined. Sections P1 to P3 are sequentially defined at an interval of 30 seconds. In addition, a section including the sections P1 and P2 is defined as a section P4 and a section including the sections P2 and P3 is defined as a section P5. A change in the value of the sleep stage shown in Fig. 20 is an illustrative example, but is not related to the actual data. In addition, the observation values of the abdominal breathing a, the chest breathing b, and the sleep stage d shown in Fig. 21 are obtained.

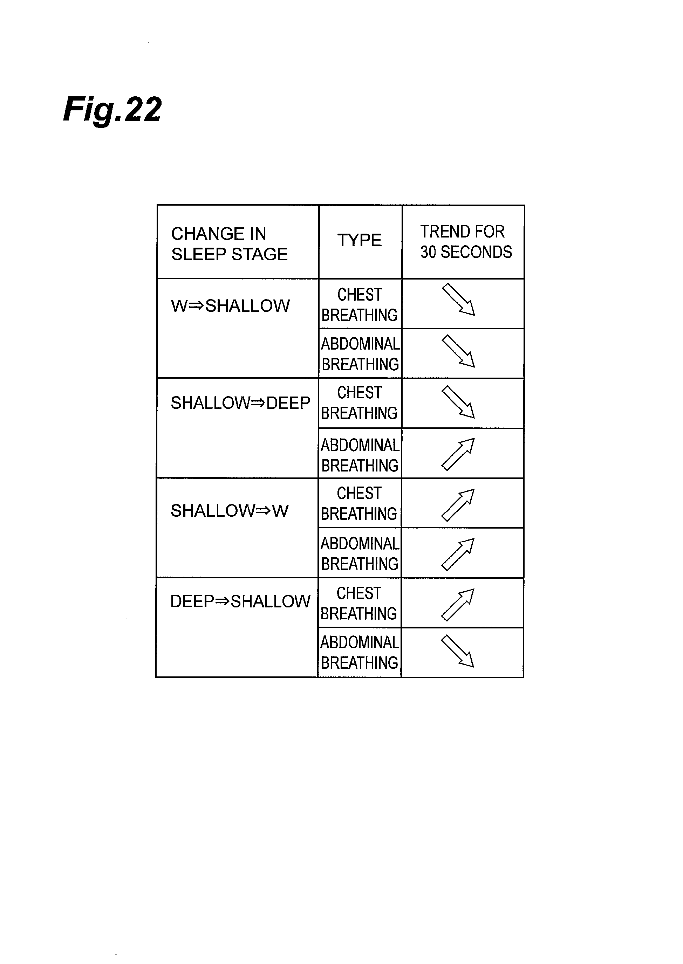

As a trend for every 30 seconds from Fig. 21, when the sleep state changes from the awakening state to the shallow sleep state, both the abdominal breathing a and the chest breathing b tend to fall, as shown in Fig. 22. When the sleep state changes from the shallow sleep state to the deep sleep state, the chest breathing b tends to fall and the abdominal breathing tends to rise. When the sleep state changes from the shallow sleep state to the awakening state, both the abdominal breathing a and the chest breathing b tend to rise. When the sleep state changes from the deep sleep state to the shallow sleep state, the chest breathing b tends to rise and the abdominal breathing tends to fall.

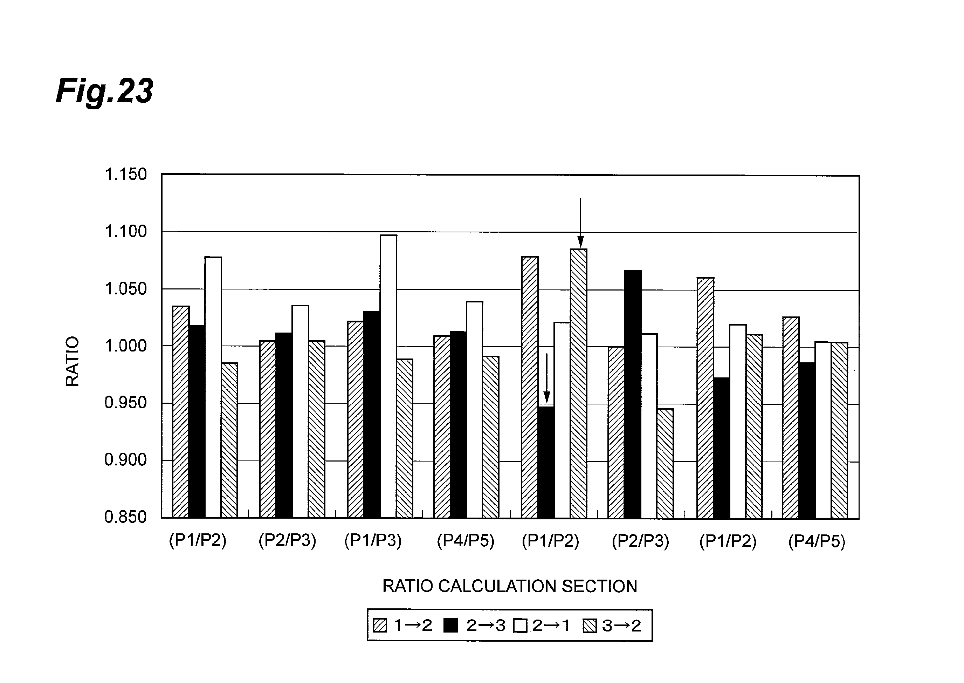

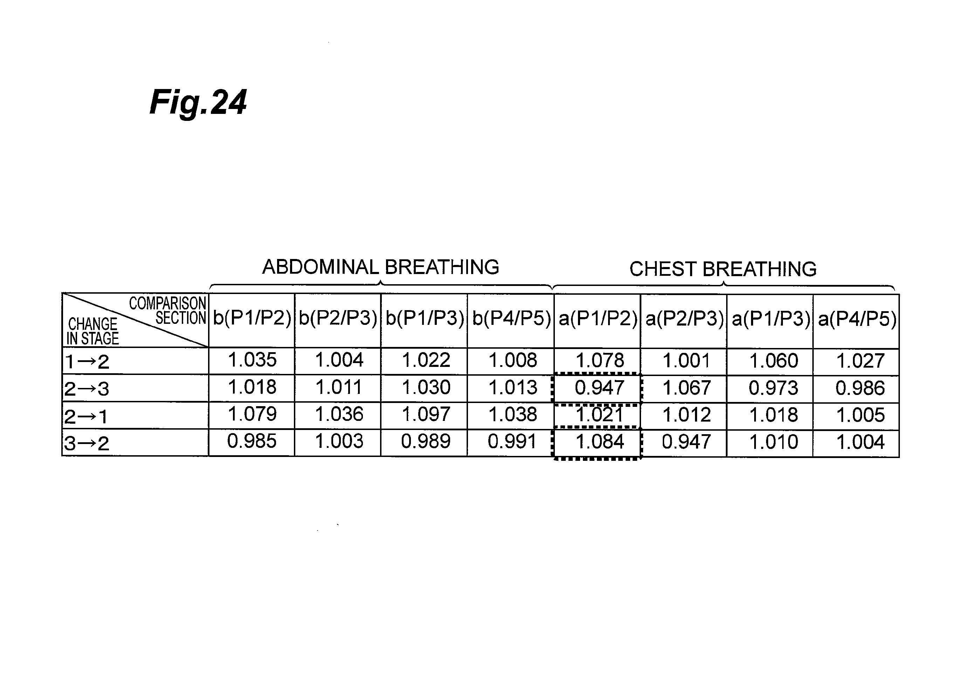

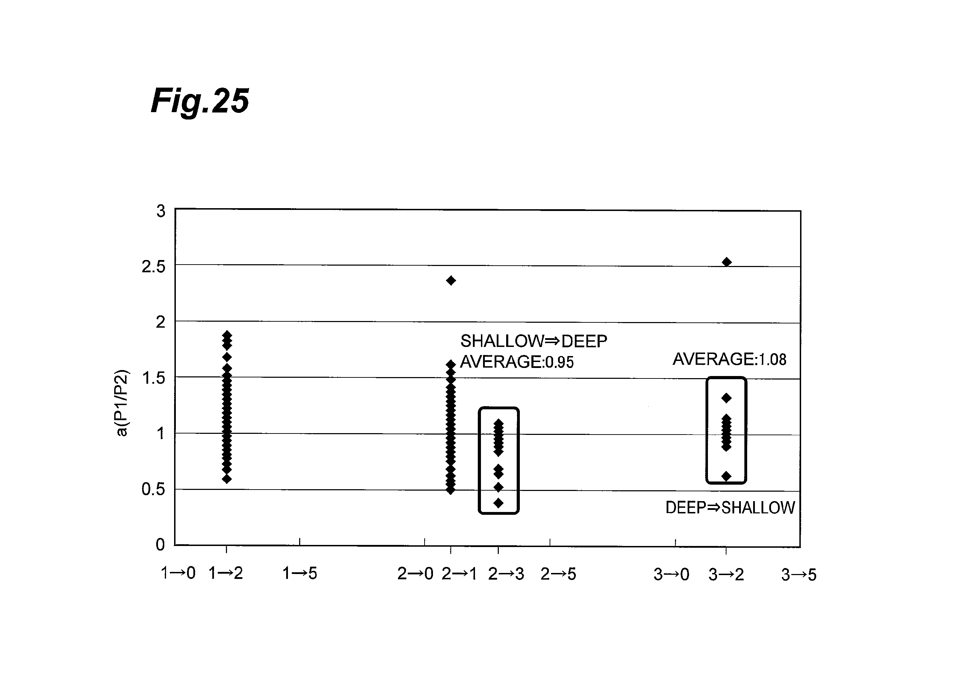

Fig. 23 is a graph illustrating a variation in the ratio of the abdominal breathing a and the chest breathing b for each of the sections P1 to P5 defined in Fig. 20 and Fig. 24 is a table illustrating values. In Fig. 23, the ratio of the section P2 to the section P1 in chest breathing is represented by b(P1/P2) and the ratio of the section P2 to the section P3 in abdominal breathing is represented by a(P2/P3). Fig. 25 shows the ratio of the section P1 to the section P2 in abdominal breathing for each variation in the sleep stage.

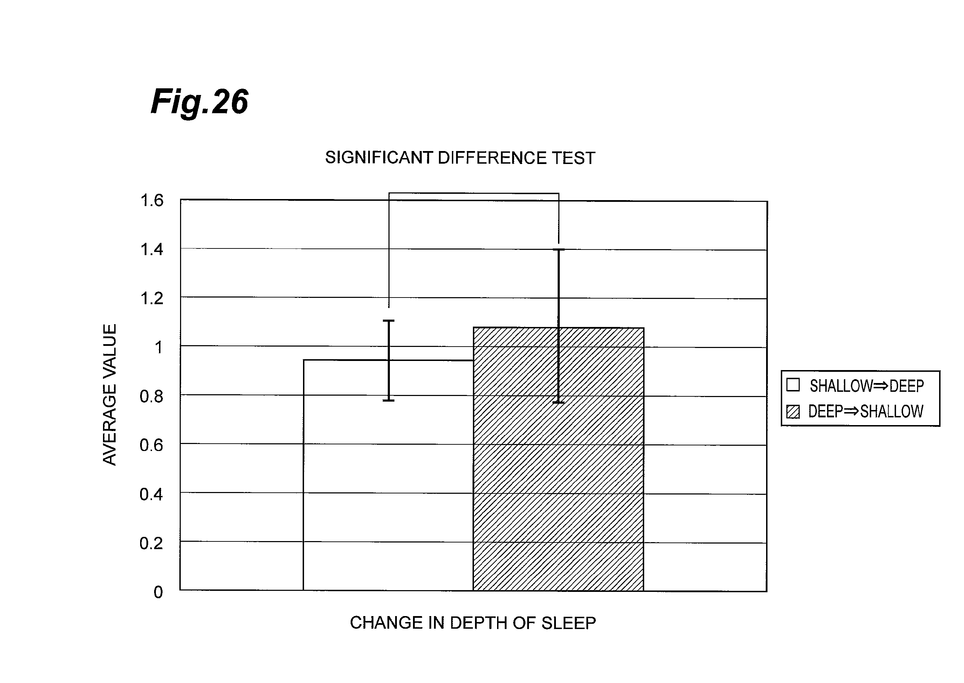

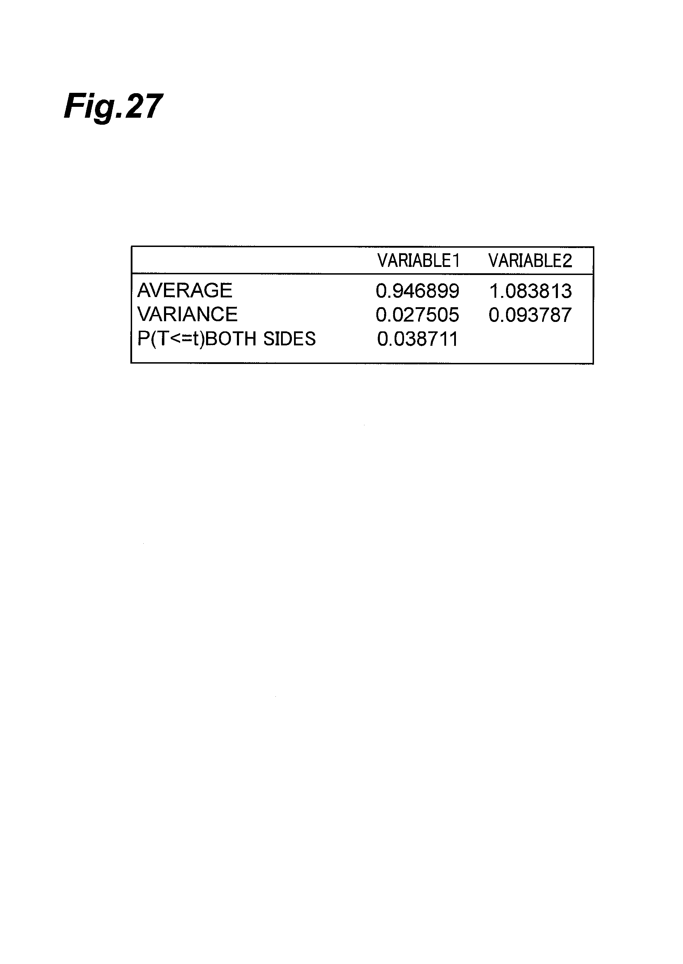

A statistically-significant difference test is performed for the ratio (a(P1/P2)) of the section P1 to the section P2 in abdominal breathing in a case in which the sleep stage changes from the shallow sleep state to the deep sleep state and a case in which the sleep stage changes from the deep sleep state to the shallow sleep state. As shown in Figs. 26 and 27, for the ratio (a(P1/P2)) of the section P1 to the section P2 in abdominal breathing, an average value of about 0.95 and a variance of 0.028 were obtained when the sleep stage changed from the shallow sleep state to the deep sleep state and an average value of about 1.08 and a variance of 0.094 were obtained when the sleep stage changed from the deep sleep state to the shallow sleep state. The data is significant at a level of 0.05.

As can be seen from the above, in this embodiment, it is possible to simply estimate the sleep stage from a change in abdominal breathing and chest breathing. A change in a breathing method, for example, the ratio (abdominal breathing/chest breathing) of abdominal breathing to chest breathing is used to check whether the sleep stage is a deep sleep state or the other states. In addition, the variance (standard deviation) of the ratio (abdominal breathing/chest breathing) of abdominal breathing to chest breathing is used to improve the accuracy of separation between an awakening state and a sleep state in the sleep stage.

Since a change in a heart beat is dominated by the autonomic nerve which originates in the brain stem, it is difficult for the object person to control the heart beat at his or her own will. Therefore, frequency analysis, such as fine fluctuation analysis, is needed in order to estimate the body motion of the object person and is not suitable for instant processing. In contrast, according to this embodiment, the arithmetic unit 30 of the sleep state estimation device 1 estimates the state of the object person on the basis of the identity of each cycle of the breathing waveform of the object person M as the feature amount of the breathing waveform. The object person M can control a change in breathing at his or her own will. Therefore, when the state of the object person M is estimated on the basis of the identity of each cycle of the breathing waveform of the object person M, it is possible to easily classify the body motions of the object person M in detail and easily improve the accuracy of estimating the state of the object person M, such as the depth of sleep or a body motion.

According to this embodiment, the arithmetic unit 30 of the sleep state estimation device 1 estimates the state of the object person M on the basis of at least one of the reproducibility, which is a fluctuation in the minimum value in each cycle of the breathing waveform, and the autocorrelation, which is the identity between the waveform shifted from the breathing waveform by an arbitrary period of time and the original breathing waveform as the identity of each cycle of the breathing waveform of the object person M. Therefore, it is possible to improve the accuracy of estimating the state of the object person, such as the depth of sleep or a body motion, with a simple process.

According to this embodiment, the arithmetic unit 30 of the sleep state estimation device 1 estimates the state of the object person M on the basis of the cycle, amplitude, autocorrelation, and reproducibility of the breathing waveform as the feature amounts of the breathing waveform of the object person M. Therefore, since four indexes, such as the cycle, amplitude, autocorrelation, and reproducibility of the breathing waveform, are combined with each other to estimate the state of the object person M, it is possible to further improve the accuracy of estimating the state of the object person M, such as the depth of sleep or a body motion.

According to this embodiment, when it is detected that the amplitude, autocorrelation, and reproducibility of the breathing waveform are changed as compared to those in the normal state of the object person M, the arithmetic unit 30 of the sleep state estimation device 1 presumes that the object person M is reseated. The inventors found that, when the object person M was reseated, the amplitude, autocorrelation, and reproducibility of the breathing waveform of the object person M were changed. Therefore, when it is detected that the amplitude, autocorrelation, and reproducibility of the breathing waveform are changed as compared to those in the normal state of the object person M, the arithmetic unit 30 presumes that the object person M is reseated. As a result, it is possible to accurately presume that the object person M is reseated.

When it is detected that the amplitude and reproducibility of the breathing waveform are changed as compared to those in the normal state of the object person, the arithmetic unit 30 of the sleep state estimation device 1 presumes that the object person M stretches the hands upward. The inventors found that, when the object person M stretched the hands upward, the amplitude and reproducibility of the breathing waveform of the object person M were changed. Therefore, when it is detected that the amplitude and reproducibility of the breathing waveform are changed as compared to those in the normal state of the object person M, it is presumed that the object person M stretches the hands upward. As a result, it is possible to accurately presume that the object person M stretches the hands upward.

When it is detected that the autocorrelation and reproducibility of the breathing waveform are changed as compared to those in the normal state of the object person M, the arithmetic unit 30 of the sleep state estimation device 1 presumes that the object person M has a conversation. The inventors found that, when the object person M had a conversation, the autocorrelation and reproducibility of the breathing waveform of the object person M were changed. Therefore, when it is detected that the autocorrelation and reproducibility of the breathing waveform are changed as compared to those in the normal state of the object person M, it is presumed that the object person M has a conversation. As a result, it is possible to accurately presume that the object person M has a conversation.

According to this embodiment, when it is detected that the cycle and amplitude of the breathing waveform are changed as compared to those in the normal state of the object person M, the arithmetic unit 30 of the sleep state estimation device 1 presumes that the object person M takes a deep breath. The inventors found that, when the object person M took a deep breath, the cycle and amplitude of the breathing waveform of the object person M were changed. Therefore, when it is detected that the cycle and amplitude of the breathing waveform M are changed as compared to those in the normal state of the object person M, it is presumed that the object person M takes a deep breath. As a result, it is possible to accurately presume that the object person M takes a deep breath.

In this embodiment, the arithmetic unit 30 of the sleep state estimation device 1 compares the feature amounts of the breathing waveform and the threshold values set to each feature amount to estimate the state of the object person M. Therefore, it is possible to estimate the state of the object person M, such as the depth of sleep or a body motion, with a simple process.

In this embodiment, the arithmetic unit 30 of the sleep state estimation device 1 sets the threshold values to each feature amount of each object person M. Therefore, it is possible to estimate the state of the object person M according to the physical constitution or taste of each object person M.

In this embodiment, the arithmetic unit 30 of the sleep state estimation device 1 estimates the state of the object person M in the vehicle, and estimates the state of the object person M while discriminating between the behavior of the vehicle and the body motion of the object person M on the basis of the acceleration of the vehicle. Therefore, it is possible to accurately estimate the state of the object person M in the vehicle while discriminating between the behavior of the vehicle and the body motion of the object person.

In this embodiment, the arithmetic unit 30 of the sleep state estimation device 1 determines whether the breathing type of the object person M is abdominal breathing or chest breathing from the breathing waveform of the object person M and estimates the depth of sleep of the object person M. Since whether the breathing type is abdominal breathing or chest breathing is closely related to the depth of sleep of the object person M, it is possible to improve the accuracy of estimating the depth of sleep.

The embodiment of the invention has been described above, but the invention is not limited to the above-described embodiment. Various modifications of the invention can be made.

### Industrial Applicability

According to the sleep state estimation device of the invention, it is possible to easily classify the body motions of the object person in detail and easily improve the accuracy of estimating the state of the object person, such as the depth of sleep or a body motion. Therefore, it is possible to execute various application programs for the object person according to, for example, the depth of sleep or the type of body motion of the object person which is estimated in detail, which makes it easy to lead the object person to a comfortable state.

### Reference Signs List

- 1:: SLEEP STATE ESTIMATION DEVICE
- 10:: SEAT
- 12:: BREATHING SENSOR
- 13a, 13b, 13c:: PRESSURE SENSOR
- 14:: ACCELERATION SENSOR
- 16, 18:: BREATHING BAND
- 20:: I/F
- 30:: ARITHMETIC UNIT
- 40:: DB

## Claims

1. A sleep state estimation device comprising:
a state estimation unit that estimates a state of an object person on the basis of identity of each cycle of a breathing waveform of the object person as a feature amount of the breathing waveform.

2. The sleep state estimation device according to claim 1,
wherein the state estimation unit estimates the state of the object person on the basis of at least one of reproducibility, which is a fluctuation in a minimum value in each cycle of the breathing waveform, and autocorrelation, which is identity between a waveform which is shifted from the breathing waveform by an arbitrary period of time and the original breathing waveform as the identity of each cycle of the breathing waveform of the object person.

3. The sleep state estimation device according to claim 2,
wherein the state estimation unit estimates the state of the object person on the basis of the cycle, an amplitude, the autocorrelation, and the reproducibility of the breathing waveform as the feature amounts of the breathing waveform of the object person.

4. The sleep state estimation device according to claim 3,
wherein the state estimation unit presumes that the object person is reseated when it is detected that the amplitude, the autocorrelation, and the reproducibility of the breathing waveform are changed as compared to those in a normal state of the object person.

5. The sleep state estimation device according to claim 3 or 4,
wherein the state estimation unit presumes that the object person stretches hands upward when it is detected that the amplitude and the reproducibility of the breathing waveform are changed as compared to those in the normal state of the object person.

6. The sleep state estimation device according to any one of claims 3 to 5,
wherein the state estimation unit presumes that the object person has a conversation when it is detected that the autocorrelation and the reproducibility of the breathing waveform are changed as compared to those in the normal state of the object person.

7. The sleep state estimation device according to any one of claims 3 to 6,
wherein the state estimation unit presumes that the object person takes a deep breath when it is detected that the cycle and the amplitude of the breathing waveform are changed as compared to those in the normal state of the object person.

8. The sleep state estimation device according to any one of claims 1 to 7,
wherein the state estimation unit compares the feature amount of the breathing waveform with a threshold value which is set to each feature amount to estimate the state of the object person.

9. The sleep state estimation device according to claim 8,
wherein the state estimation unit sets the threshold value to each feature amount of each object person.

10. The sleep state estimation device according to any one of claims 1 to 9,
wherein the state estimation unit estimates the state of the object person in a vehicle and estimates the state of the object person while discriminating between a behavior of the vehicle and a body motion of the object person on the basis of acceleration of the vehicle.

11. The sleep state estimation device according to any one of claims 1 to 10,
wherein the state estimation unit determines whether the breathing type of the object person is abdominal breathing or chest breathing from the breathing waveform of the object person to estimate the depth of sleep of the object person.
